# EUROPEAN PATENT SPECIFICATION

(11) **EP 3 880 383 B1**
(45) Date of publication and mention of the grant of the patent: **01.11.2023**
(21) Application number: 19809569.7
(22) Date of filing: 15.11.2019
(51) Int. Cl.: A24F 40/70, B21F 3/04

(54) **VAPE TOOL AND VAPE TOOL KIT FOR MAKING VAPING COILS**
VERDAMPFERINSTRUMENT UND VERDAMPFERINSTRUMENTENSATZ ZUR HERSTELLUNG VON VERDAMPFUNGSSPULEN
OUTIL POUR VAPORISATEUR ET TROUSSE D'OUTILS POUR VAPORISATEUR POUR LA FABRICATION DE SPIRES DE VAPOTAGE

(30) Priority: 15.11.2018 GB 201818614
(43) Date of publication of application: 22.09.2021
(73) Proprietor: Graph-X Ltd, Halesowen Birmingham B62 8EX (GB)
(72) Inventor: SHEPPARD, Christopher, Birmingham B62 8EX (GB); DRINKWATER, Benjamin, Birmingham B62 8EX (GB)
(74) Representative: Maidment, Marc
(86) International application number: PCT/GB2019/053240
(87) International publication number: WO 2020/099884

(56) References cited:
- US-A- 3 378 906
- US-A- 3 755 872
- US-A- 3 994 320
- US-A- 5 245 749

## Description

The present invention relates to a vape tool and vape tool kit for making vaping coils.

### BACKGROUND TO THE INVENTION

Vaping is a highly popular recreational activity, which is being favoured over smoking. It is estimated by the World Health Organisation that the number of people vaping by the end of 2016 had grown to around 35 million people, from 7 million in 2011. It is known to provide tool kits for making vaping coils for vaping, and making coils is regarded by many as a fun and creative part of the vaping experience.

A problem with current vape tool kits is that they are quite large and tend to be provided in soft shell cases, with zip fastener extending around the periphery of the casing. The tool kits take the appearance of old-fashioned soft-shell pencil cases and tools, such as pliers, tweezers, cutters, screwdrivers and coiling rods are held in place by elastic.

Specific problems arise in the storing of cotton, which must be kept dry and clean. Furthermore, the vaping wire can easily become unravelled and damaged, since it is supplied on open reels. It is also often wound by hand around a coiling rod or the stem of a screwdriver. More sophisticated kits include coiling rods with apertures for securing an end of the wire whilst the wire is rotated around the rod, and some include wire guides for guiding the wire being coiled.

It is an object of the present invention to provide a vape tool and vape tool kit which reduces or substantially obviates the aforementioned problems.

### STATEMENT OF INVENTION

According to the present invention there is provided a vape tool comprising a coiling rod, and a coiling tool for engaging with and rotating around the coiling rod, the coiling tool having a housing with a substantially conical inner wall, a collet disposed inside the housing and arranged to bear against the inner wall, an internal diameter of the collet being determined by the longitudinal position of the collet within the housing and a control means for controlling the longitudinal position of the collet within the housing.

The tool provides a simple and convenient way of forming coils of the same size and quality. Furthermore, the collet arrangement can accept different diameters of coiling rod.

The control means may be a thumb screw threadingly engaged with the housing and connected to the collet. An aperture may be provided in an end of the housing for receiving the coiling rod.

A coiling rod holder may be provided for use with the coiling rod and coiling tool. The coiling rod holder may have a body which is separate to or discrete from the other components of the vape tool. The coiling rod holder may be of similar size to the coiling tool. This allows the coiling assembly (tool, rod and rod holder) to be more easily held or manually manipulated when making a coil. The coiling rod holder may be a cylinder or may be substantially cylindrical for ease of rotation during use.

A coiling rod aperture may be provided in an end of the coiling rod holder for receiving the coiling rod. The coiling rod aperture may include a substantially hexagonal or non-circular portion for receiving a substantially hexagonal or non-circular end of the coiling rod.

A first hole for guiding coiling wire may be provided adjacent to the coiling rod aperture. A first corresponding hole may be provided on a side or another end of the coiling rod holder. The two holes may be connected together (e.g. via a bore) through the body of the coiling rod holder. The bore should be at least as wide as the diameter of the coiling wire.

A second hole may be provided adjacent to the coiling rod aperture. The second hole may be on the opposite side of the coiling rod aperture to the first hole. A second corresponding hole may be provided on a side or another end of the coiling rod holder. The two second holes may be connected together (e.g. via a bore) through the body of the coiling rod holder. This means that the coiling rod holder is suitable for use in either hand.

At least one slot or notch or cut-out may be provided across the top of the coiling rod holder. The slot may be offset to one side of the coiling rod aperture. The slot may extend into the holder on a plane or axis which is substantially parallel to the axis of the coiling rod aperture. In some embodiments, two slots may be provided on opposite sides of the top of the coiling rod holder for left or right-handed use. The slot(s) can receive and guide flat ribbon (or ribbon-type wire) when making a coil from the ribbon.

The coiling rod holder may be magnetic or magnetised. A magnet or magnets may disposed in the coiling rod holder. The magnet(s) may be disposed adjacent to the coiling rod aperture. The magnet(s) may be disposed at or adjacent to the base of the coiling rod aperture. Where the coiling rod is magnetic, or made of (or includes) a magnetic or magnetisable material, the coiling rod can be held more securely in the coiling rod holder. Where a coil is tightened too much, having the coiling rod secured in the holder by a magnet means that the rod can be more easily withdrawn from the coil, instead of the rod being clamped within the coil and trapped in situ within the coil.

A wire guide may be disposed at an end of the housing for guiding wire around the coiling rod on rotation of the coiling tool relative to the coiling rod. The wire guide may be a screw.

The vape tool may be part of a kit including a casing. The casing may include a central body portion, an upper end cap and a lower end cap. The upper end cap may be securable to one end of the central body portion. The lower end cap may be securable to the other end of the central body portion.

One or both of the upper and lower end caps may include knurling or longitudinal slots on an outer side of that end cap, for improving grip when screwing or unscrewing the end cap.

A spool for storing vaping wire may be disposed within the central body portion and/or lower end cap.

The vape tool may be part of a kit including a casing, the casing having a central body portion, an upper end cap securable to one end of the central body portion, an intermediate cap securable to the other end of the central body portion and a lower end cap securable to the intermediate cap. Conveniently the casing may be cylindrical and sized to fit in a pocket.

An aperture may be provided in the intermediate cap for receiving a coiling rod. The end of the coiling rod received in the intermediate cap may be hexagonal and the aperture in the intermediate cap may be correspondingly hexagonal.

Advantageously one end of the coiling rod can be located in the intermediate cap, whilst the other end is located in the coiling tool. The hexagonal engagement serves to prevent rotation of the rod relative to the cap. It will be appreciated that other non-circular shapes may be provided for the end of the coiling rod and rod holder or portion of the intermediate cap. A tool barrel for storing tools may be disposed within the central body of the casing.

A spool for storing vaping wire may be disposed within the central body portion and intermediate cap. The spool may have external protrusions spaced around the spool for retaining a winding of vaping wire.

A sealable area for containing cotton may be formed within the lower end cap. The sealable area may be partitioned to provide a space for cotton and a space for screws and pre-made vaping coils.

According to a second aspect of the invention there is provided a vape tool kit comprising a casing including a central body portion and an upper end cap attachable to one end of the central body portion and enclosing a first space, an intermediate cap enclosing a second space, and a lower end cap enclosing a third space, a tool kit being located in the first space, a spool and sealable cotton storage area being located in the second and third spaces, in which the intermediate cap is attachable to the other end of the central body portion and the lower end cap is attachable to the intermediate cap, or in which the intermediate cap is disposable in the lower end cap and the lower end cap is attachable to the central body portion.

Advantageously the vape tool kit is modular, in that a number of cylindrical elements are connected together. It is envisaged that the tool kit could be extended, for example, by adding one or more further modules. For example, a further intermediate module could be positioned between the central body portion and the upper end cap.

The tool kit may include a cylindrical barrel, having apertures formed therein for receiving tools. The tool barrel may be made from silicone rubber. The tool barrel may be disposed within the first space.

The spool may be formed from upper and lower parts and may include a U-shaped circular wall onto which wire can be coiled. The spool may have a cover element which may lie over the U-shaped circular wall for protecting coiled wire on the spool.

External protrusions may be provided on the spool for temporarily receiving a second coil of wire.

A cover or cover plate may close a space in the lower end cap and may form the sealable cotton storage area. The cover or cover plate may be provided with a peripheral seal.

The upper end cap may be screwed to one end of the central body portion. The intermediate cap may be screwed to the other end of the central body portion. The lower end cap may be screwed to the intermediate cap. In some instances, the lower end cap may be screwed to another end of the central body portion. The intermediate cap may be disposed inside the lower end cap.

According to a third aspect of the invention there is provided a vape tool kit comprising a casing including a central body portion and an upper end cap attachable to one end of the central body portion and enclosing a first space, an intermediate cap attachable to the other end of the central body portion and enclosing a second space, and a lower end cap attachable to the intermediate cap and enclosing a third space, a tool kit being located in the first space, a spool and sealable cotton storage area being located in the second and third spaces.

Any feature or combination of features presented with respect to the first, second or third aspects of the invention may be included in any of the other aspects of the invention.

### BRIEF DESCRIPTION OF THE DRAWINGS

For a better understanding of the present invention, and to show more clearly how it may be carried into effect, reference will now be made by way of example only to the accompanying drawings, in which:
Figure 1 shows a cross sectional view of a tool for making vaping coils;
Figure 2 shows an upper end of a tool cartridge forming part of the tool of Figure 1;
Figure 3 shows the lower end of the tool cartridge of Figure 2;
Figure 4A shows a spool forming part of the tool of Figure 1;
Figure 4B shows the spool of Figure 1 in a disassembled condition for loading with wire;
Figure 5 shows a lower end cap of the tool of Figure 1;
Figure 6 shows a cross sectional side view of the manufacture of a vaping coil using a collet clamp and coiling rod of the tool of Figure 1;
Figure 7 shows a part perspective, part cross sectional view of the collet clamp and coiling rod of Figure 6;
Figure 8 shows a perspective view of a coiling rod holder;
Figure 9 shows a cross-sectional side view of the coiling rod holder of Figure 8 engaged with a coiling rod and coiling tool;
Figure 10 shows a perspective view of the top side of a cover for the lower end cap; and
Figure 11 shows another perspective view of the cover of Figure 11.

### DESCRIPTION OF PREFERRED EMBODIMENTS

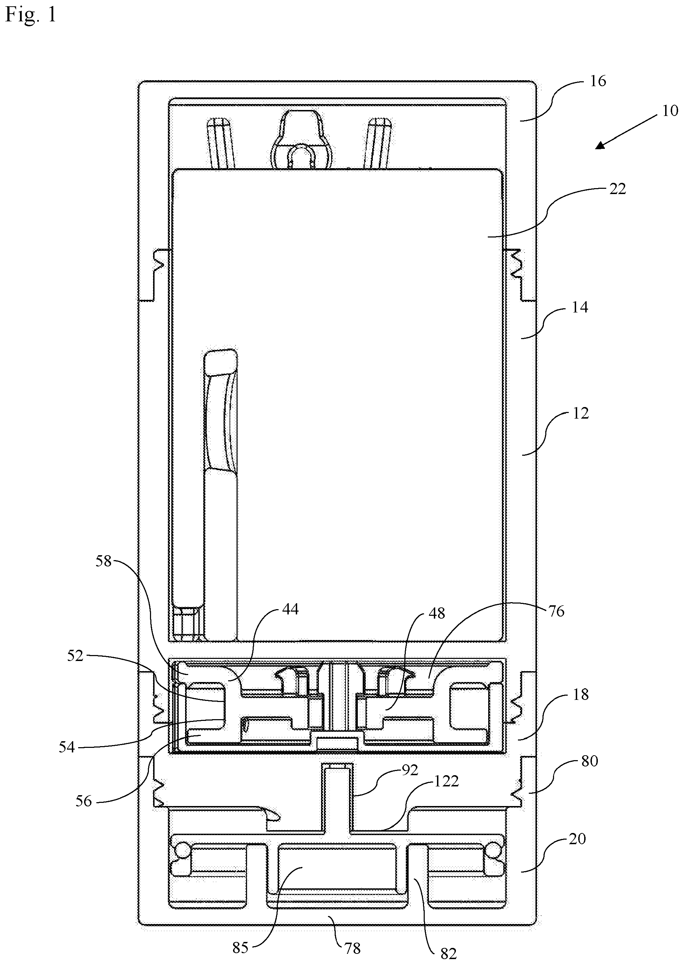

Referring firstly to Figure 1, a vape tool kit is indicated generally at 10. The kit 10 is enclosed in a housing 12. The housing 12 is cylindrical and is designed to fit in a person's pocket. In one embodiment, it is 150mm in length with a diameter of 55mm. The housing 12 is made from metal, preferably anodised aluminium, and is waterproof, to protect the contents. A rigid plastic may also be utilised for the housing 12. In other words, it is completely sealed to prevent ingress of dirt and/or moisture.

The housing has a central body portion 14, an upper end cap 16, an intermediate cap 18 and a lower end cap 20. The central body portion 14 and upper end cap 16 define a cavity, which houses a tool holder or barrel 22, preferably made from a firm silicone. The tool barrel 22 fits snugly within the housing 12, but can be removed, when the upper end cap 16 is detached from the central body portion.

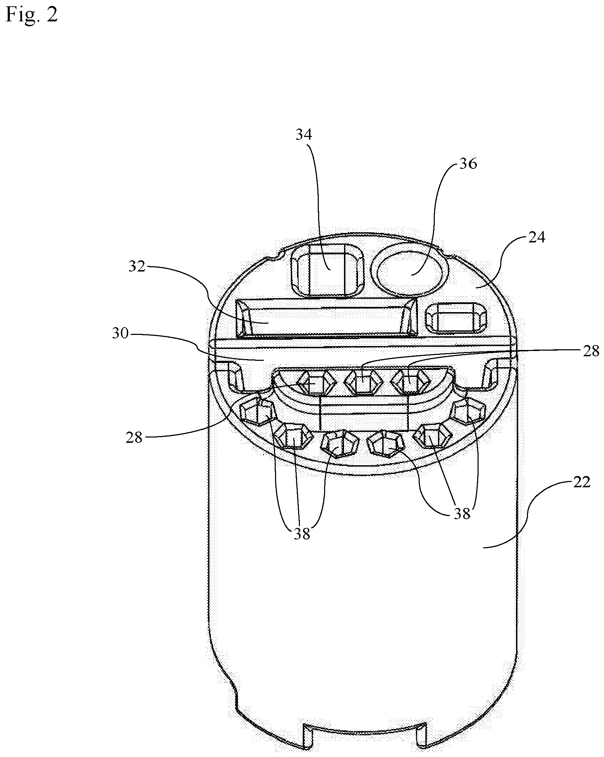

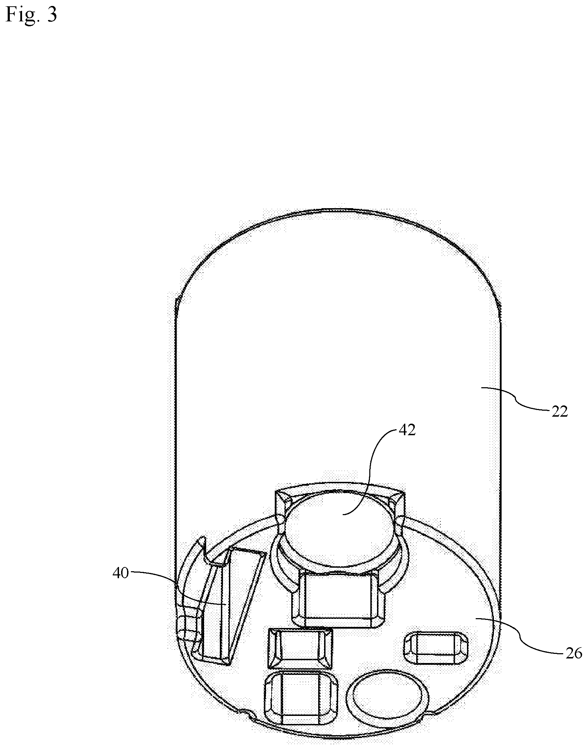

Referring also to Figures 2 and 3, the tool barrel 22 is provided with a number of tool apertures in the upper end 24 and the lower end 26. In the upper end 24, there are three apertures 28 for flat head, hexagon and cross head screwdriver bits, a slot 30 for a pliers, a slot 32 for wire cutters, an aperture 34 for ceramic or aluminium tweezers, an aperture 36 for a screwdriver, and a plurality of apertures 38 for different diameter coiling rods. A wire brush can also be located in one of the apertures 38, and if necessary, the shape of the aperture can be altered to have a substantially circular cross-section accordingly. A wire brush is typically used to clean the contacts of a vaping device.

Some of the apertures extend through the barrel 22, but in the lower end 26 of the tool barrel 22, there is an aperture 40 for receiving scissors, and an aperture 42 for a coiling tool. The scissors may be collapsible or foldable.

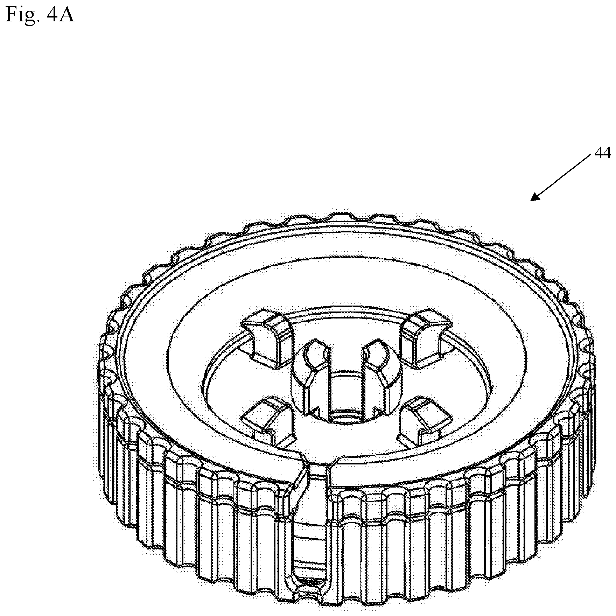

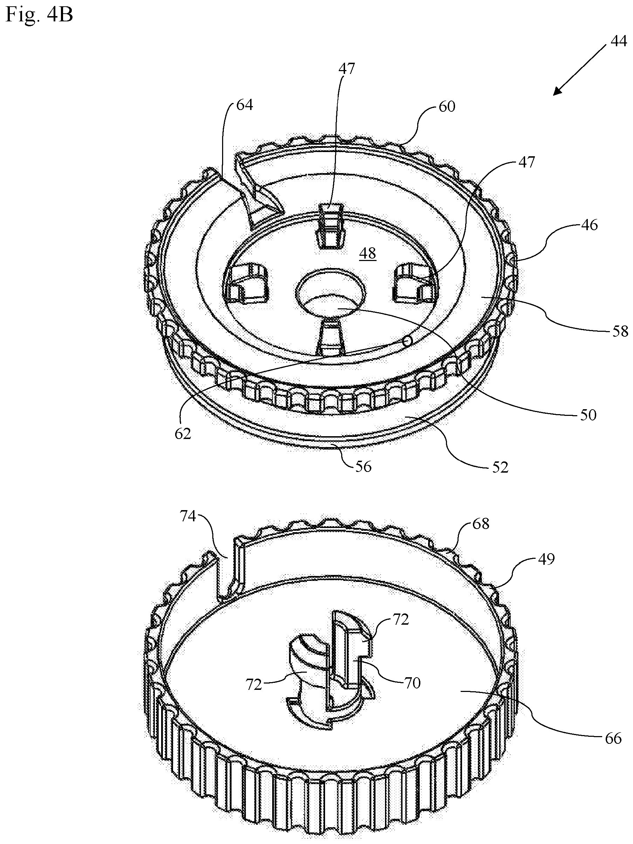

Referring to Figures 4A and 4B, a spool for vaping wire is indicated at 44. The spool 44 can also be seen in cross-section in Figure 1. The spool 44 is formed from 2-parts, a spool element 46 and a cover element 48. The spool element 46 includes a central circular plate 48, with a circular aperture 50 at its centre. A circular wire holder 52 extends around the periphery of the circular plate and is substantially U-shaped. The U-shape includes an inner peripheral wall 54, a lower wall 56 and an upper wall 58. The upper wall 58 extends beyond the lower wall 56, and is provided with a grip portion 60 around its outer edge, formed by part circular recesses. An aperture 62 is provided through the upper wall 58 to a position adjacent the inner wall 54 for retaining the end of a wire to be wound on the coil.

Four hook-shaped protrusions 47 are equi-spaced around the circular plate 48, that is at 90 degrees apart. They terminate below the plane of the upper wall 58. The hooks face outwardly, and are positioned closer to the back of the inner peripheral wall 54 then the aperture 50. A slot 64 extends radially through part of the inner peripheral wall 54 and upper wall 58, disposed on one side of the circular plate 48.

The cover element 49 comprises a circular plate 66 with a circumferential flange 68. A resilient spigot 70 extends from the centre of the circular plate 66, to the same side as the flange 68. The resilient spigot 70 is not solid, but has a slot along part of its length to enable inward flexing. Protrusions 72 are provided at the end of the spigot 70, which are chamfered at their distal end to enable pushing through the aperture 50 of the spool element 46 and snap engagement against the circular plate 48, best seen in Figure 4A.

A slot 74 is provided through the circumferential flange 68 and can be arranged to align with the slot 64, when the spool is assembled. The use of the spool 44 is described further below.

The spool 44 is located and stored in a space 76 located between the central body portion 14 and the intermediate cap 18. The space 76 is cylindrical and the spool 44 fits snugly within the space.

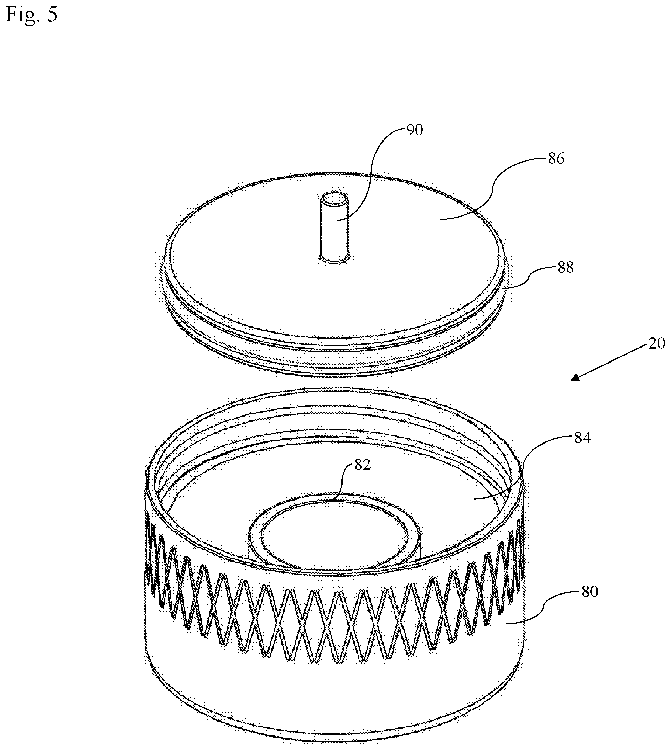

Referring now to Figures 1 and 5, the lower end cap 20 forms a cotton storage compartment. The lower end cap 20 has an end wall 78 adjoining a peripheral wall 80. Inside the end cap 20, a circular wall 82, concentric with the peripheral wall 80 extends upwardly for around half the height of the cap. This defines a space 84, in the form of a hoop between the peripheral wall 82 and inner circular wall 82, where cotton can be wound and stored. A circular cover plate 86 closes the space 84 and has a seal 88, preferably a nitrile seal, around its periphery. The seal prevents ingress of moisture and/or dirt and keeps the cotton clean. A space 85, inside the circular wall 82 is used to store screws and pre-formed coils. A handle 90, in the form of a cylindrical spigot, extends outwardly from the centre of the cover plate 86. When the lower end cap 20 is located on the intermediate cap 18, as shown in Figure 1, the spigot 90 locates in a recess 92, disposed on the underside of the intermediate cap. The recess is hexagonal in cross-section, as described further below.

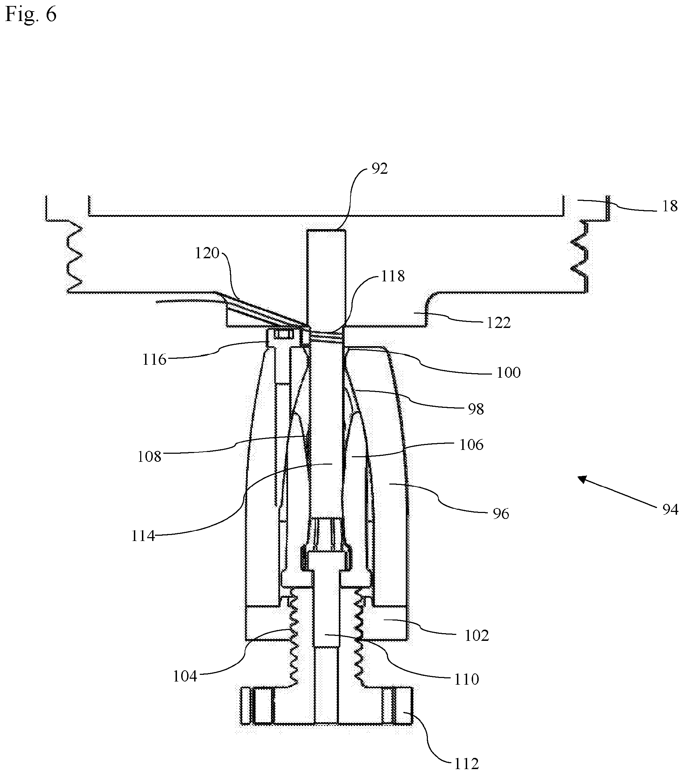

A coiling tool 94 will now be described with reference to Figure 6. The coiling tool 94 is a clamping device utilising a collet design for holding coiling rods of different diameters. The coiling tool 94 has a housing 96 with a substantially conical inner wall 98. One end of the housing 96 is open, at 100, and the other end is closed by a closure plate 102. A screw threaded aperture 104 is provided through the closure plate 102. A resilient collet 106 with central bore 108 sits within the housing 96. A pin 110 with enlarged head seated in a back end of the collet 106 is connected to a thumb screw 112, threadingly engaged in the screw threaded aperture 104. The end of the collet 106 is held to the end of the thumbscrew 112 by the pin 110.

As the thumbscrew 112 is turned to advance it towards the collet 106, the collet is pushed into the reducing diameter of the inner wall and the central bore 108 of the collet reduces for clamping a coiling rod, an example of which is indicated at 114. As the thumbscrew 112 is turned in the other direction, the collet is drawn back into the housing and the central bore 108 enlarges, thereby freeing the coiling rod.

The other end of the coiling rod 114 is non-circular, in this case hexagonal, and locates in the aperture 92 on the underside of the intermediate cap 18. A threaded screw 116 is threaded into an aperture in the end of the housing 96, to one side of the central aperture. The head of the screw 116 acts as a guide for vaping wire 118 being coiled, as described further below. A hole 120 is provided through an upstand in the underside of the intermediate cap 18, which leads from the side of the upstand to a position adjacent the aperture 92 for the coiling rod 114. The hole 120 also serves to guide coiling wire.

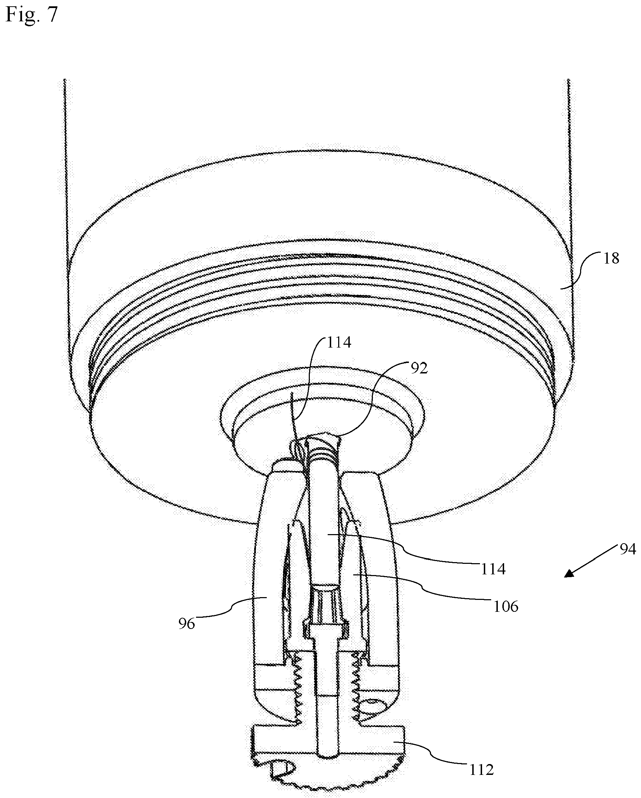

The vape tool kit 10 can be carried easily in a person's pocket. The use of the tool kit 10 will now be described with reference to Figures 6 and 7. In use, the upper end cap 16 can be removed from the central body portion 14 and the tool barrel 22 removed. The tool barrel 22 has a flat base and can stand on a table or working surface. The tools can be removed and put away in the barrel as desired.

The intermediate cap 18 is then removed from the central body portion 14 and the spool 44 removed. A portion of wire can be unwound from the spool 44 for use in making coils. The wire can be temporarily wound around the hook shaped protrusions 47 on the outer of the spool 44 to keep it tidy and a short piece cut for making a coil. The spool 44 can then be replaced in the space 76 in the central body portion 14 and the intermediate cap replaced. At this point, the lower end cap 20 is removed to expose the hexagonal aperture 92.

A suitable coiling rod 114 is then selected from the tool barrel and engaged in the hexagonal aperture 92. The piece of wire 118 is fed through the aperture 120 with around 10mm of wire exposed beneath the aperture. The coiling tool 94 is then removed from the tool barrel 22 and tightened onto the exposed distal end of the coiling rod 114, as previously described using the thumb screw 112. The intermediate end cap 18, central body portion 14 upper end cap 16, and coiling rod 114 are then rotated relative to the coiling tool 94. The head of the screw 116 bears against the wire 118 and the rotation forms a coil of wire around the coiling rod 114. The rotation continues until the desired size of coil is completed. The coiling tool 94 can then be disconnected from the coiling rod 114 and the formed coil removed and trimmed.

A tester, which tests the resistance of the wire by applying a voltage across it, can also usefully be stored in the tool barrel. Before using the coil, its resistance should be tested to make sure that it is safe to use. The completed coil can then be used immediately or stored in the space 85. If the coil is utilised, then it is screwed into a vaping unit in conventional manner, liquid added and cotton fed through the coil. A coil can also be tested in situ, in order to test a coil and vape deck of a vaping device.

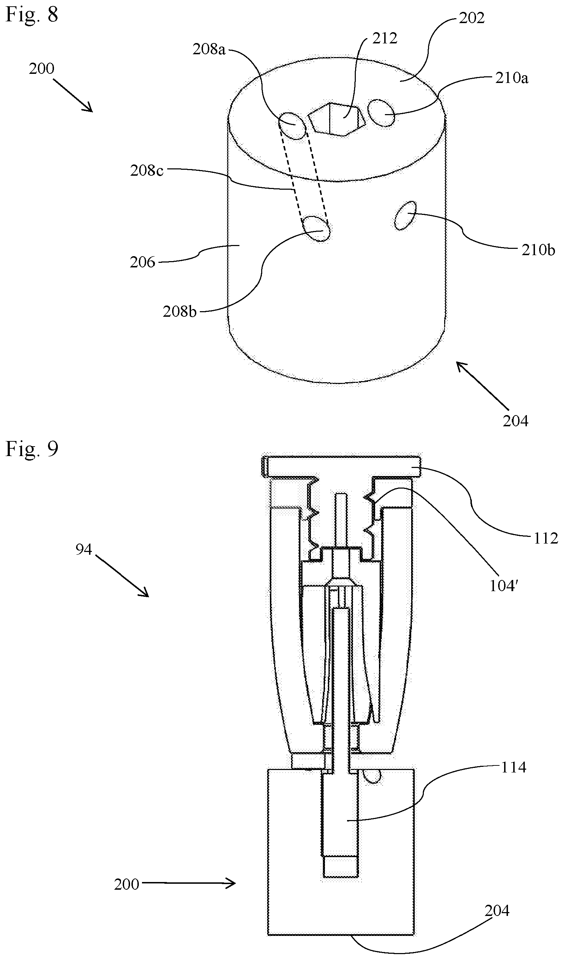

Referring also to Figures 8 to 11, components of another embodiment or other embodiments of a vape tool and vape tool kit will now be described.

A coiling rod holder is indicated generally at 200. The coiling rod holder 200 can be used as a handle for making a coil, that is to say it is handheld. The holder 200 can be controlled or manipulated with one hand whilst the coiling tool 94 can be operated or manipulated with the other hand. The coiling rod holder 200 is more easily held and wielded than the casing with the coiling rod in the intermediate cap and central body portion.

The coiling rod holder 200 is of a similar diameter to the coiling tool 94. The coiling rod holder 200 is about half the length of the coiling tool 94 in this embodiment, but it will be appreciated that the holder 200 may have any suitable length.

The coiling rod holder 200 is substantially cylindrical. The holder 200 includes a first end 202, a second end 204 and a sidewall 206. A coiling rod aperture 212 is provided in the first end. The coiling rod aperture 212 is located substantially centrally in the holder 200. The aperture 212 extends about halfway into the holder 200. The aperture has a hexagonal cross-section in this embodiment.

The holder 200 includes a first pair of holes 208a, 208b for guiding coiling wire like the hole 120 in the earlier embodiment. The holder 200 also includes a second pair of holes 210a, 210b. In the first pair of holes 208a, 208b, one hole 208a is provided in the first end 202 adjacent to the coiling rod aperture 212, and the other hole 208b is provided in the curved sidewall 206. In the second pair of holes 210a, 210b, one hole 210a is provided in the first end 202 adjacent to the coiling rod aperture 212, on the other side of that aperture 212 to the first hole 208a, and the other hole 210b is provided in the curved sidewall 206.

The holes 208a, 208b are linked by a bore (indicated at 208c by dashed lines). The bore 208c extends through the body of the holder 200. In this embodiment, the bore 208c is substantially straight. Similarly, the bore 208c is of substantially constant width in this embodiment. It will be appreciated that the bore may have any form suitable for the coiling wire to pass through. Also, although not shown, a second bore connects the holes 210a, 210b together, and is substantially similar to the bore 208c.

Figure 9 shows the coiling rod holder 200 in engagement with the coiling rod 114 and coiling tool 94. The process of making a coil using these parts is substantially similar to the embodiment described previously, with the coiling rod holder 200 being used in place of the intermediate cap 18 and other parts.

The coiling tool 94 is broadly similar to the earlier embodiment, but it will be noted that one of the differences is the screw thread (indicated at 104') for the thumbscrew 112. In this embodiment, there are far fewer turns required to withdraw or replace the thumbscrew because there are a lower number of screw thread turns per unit length along the screw-threaded portion. For example, this may be achieved by having fewer turns or an increased helix angle or increased thread pitch compared to the earlier embodiment.

In Figure 9, the screw thread 104' spirals around the thumbscrew 112. In this case, the crests of the thread 104' are relatively elongate, and longer than the thread is deep (or greater than the distance between adjacent roots on the same side of the thread 104'). About 2 to 3 turns of the thumbscrew 112 would be enough to fully withdraw or insert the thumbscrew 112.

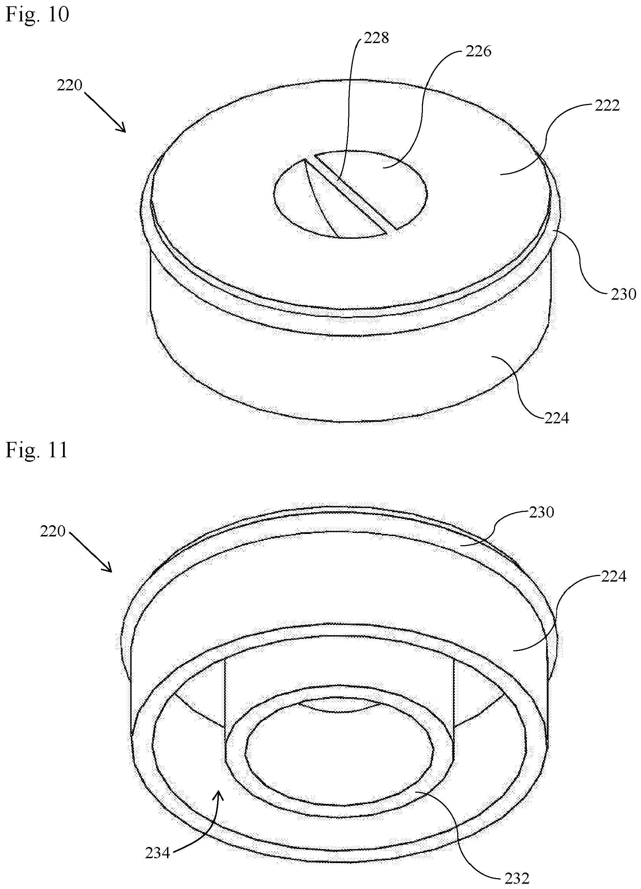

Referring also to Figures 10 and 11, a cover is indicated generally at 220. The cover 220 may be considered as a storage cap or intermediate cap which can be placed in another version of the lower end cap (not shown). Instead of providing internal structure in the lower end cap 20 and a cover plate 86, the cover 220 itself can be shaped to provide the necessary storage space for cotton, for example. This simplifies the structure of the lower end cap.

The cover 220 includes a top surface 222 and a sidewall 224 connected to the top surface 222. The sidewall 224 is curved in this embodiment. The cover 220 is approximately cylindrical for fitting within the lower end cap. A circumferential seal 230 is provided around the upper end of the sidewall 224, next to the top surface 222.

A recess 226 is provided in the top surface 222. A handle member 228 is provided across the recess 226 to allow the cover 220 to be withdrawn from or placed into the lower end cap. It will be appreciated that a spigot is not required in this instance. The intermediate cap may also be omitted in some embodiments, because there is no spigot to accommodate when assembling the parts for storage.

The handle member 228 is in the form of a divider or wall. The depth of the recess 226 and size of the wall 228 is sufficient for the wall to be grasped by a thumb and finger, or by two fingers.

A secondary wall 232 is provided within and spaced from the sidewall 224. A chamber or storage space is indicated generally at 234. The chamber 234 is defined by the inner surface of the sidewall 224, the outer surface of the secondary wall 232, and the underside of the top surface 222. Pre-formed coils and other parts may be stored in the space within the secondary wall 232 in a similar manner to the earlier embodiment.

The embodiments described above are provided by way of example only, and various changes and modifications will be apparent to persons skilled in the art without departing from the scope of the present invention as defined by the appended claims.

## Claims

1. A vape tool for the manufacture of a vaping coil comprising a coiling rod (114), and a coiling tool (94) for engaging
with and rotating around the coiling rod,
the coiling tool having a housing (96) with an inner wall, a collet (106) disposed inside the housing and arranged to bear against the inner wall, an internal diameter of the collet being determined by the longitudinal position of the collet within the housing and a control means (112) for controlling the longitudinal position of the collet within the housing,
***characterised in that*** the inner wall includes a substantially conical inner wall, and the collet is arranged to bear against the substantially conical inner wall.

2. A vape tool as claimed in claim 1, in which the control means (112) is a thumb screw threadingly engaged with the housing (96) and connected to the collet (106).

3. A vape tool as claimed in claim 1 or claim 2, in which an aperture is provided in an end of the housing (96) for receiving the coiling rod (114).

4. A vape tool as claimed in any of claims 1 to 3, in which a coiling rod holder (200) is provided for use with the coiling rod and coiling tool (94), wherein the coiling rod holder comprises a coiling rod aperture (212) disposed in an end of the coiling rod holder for receiving the coiling rod (114).

5. A vape tool as claimed in claim 4, in which a hole (208a) for guiding coiling wire is provided adjacent to the coiling rod aperture (212), and a corresponding hole (208b) is provided on a side or another end of the coiling rod holder (200) and connected to the hole through the body of the coiling rod holder.

6. A vape tool as claimed in claim 5, in which a second hole (210a) is provided adjacent to the coiling rod aperture (212), opposite the first hole (208a), and a second corresponding hole (210b) is provided on a side or another end of the coiling rod holder (200) and connected to the second hole through the body of the coiling rod holder.

7. A vape tool as claimed in any of claims 4 to 6, in which the coiling rod aperture (212) includes a substantially hexagonal or non-circular portion for receiving a substantially hexagonal or non-circular end of the coiling rod (114).

8. A vape tool as claimed in any preceding claim, in which a wire guide is disposed at an end of the housing (96), for guiding wire around the coiling rod (114) on rotation of the coiling tool (94) relative to the coiling rod, optionally in which the wire guide is a screw (116).

9. A vape tool as claimed in any preceding claim, in which the vape tool is part of a kit (10) including a casing (12), the casing having a central body portion (14), an upper end cap (16) securable to one end of the central body portion, and either:
A) a lower end cap (20) securable to the other end of the central body portion; or
B) an intermediate cap (18) securable to the other end of the central body portion and a lower end cap (20) securable to the intermediate cap.

10. A vape tool and kit as claimed in claim 9, comprising the features of (B), in which an aperture (92) is provided in the intermediate cap (18) for receiving a coiling rod (114).

11. A vape tool and kit as claimed in claim 10, in which the end of the coiling rod (114) received in the intermediate cap (18) is hexagonal and the aperture (92) in the intermediate cap is correspondingly hexagonal.

12. A vape tool and kit as claimed in any one of claims 9 to 11, in which a tool barrel (22) for storing tools including the vape tool is disposed within the central body portion (14) of the casing (12).

13. A vape tool and kit as claimed in any one of claims 9 to 12, in which a spool (44) for storing vaping wire is disposed (i) within the central body portion (14) and/or the lower end cap (20) if the kit comprises the features of (A), or (ii) within the central body portion and the intermediate cap (18) if the kit comprises the features of (B).

14. A vape tool and kit as claimed in claim 13, in which the spool (44) has external protrusions (47) spaced around the spool for retaining a winding of vaping wire.

15. A vape tool and kit as claimed in any one of claims 9 to 14, in which a sealable area for containing cotton is formed within the lower end cap (20), optionally in which the sealable area is partitioned to provide a space (84) for cotton and a space (85) for screws and pre-made vaping coils.

## Patentansprüche

1. Verdampferinstrument zur Herstellung einer Verdampferspirale, die einen Spiralstab (114) und ein Spiralwerkzeug (94) zum Eingreifen mit dem Spiralstab und Drehen um diesen umfasst,
wobei das Spiralwerkzeug ein Gehäuse (96) mit einer Innenwand, ein Spannfutter (106), das sich innerhalb des Gehäuses befindet und so angeordnet ist, dass es an der Innenwand anliegt, wobei ein Innendurchmesser des Spannfutters durch die Längsposition des Spannfutters innerhalb des Gehäuses bestimmt wird, und ein Steuermittel (112) zum Steuern der Längsposition des Spannfutters innerhalb des Gehäuses aufweist,
**dadurch gekennzeichnet, dass** die Innenwand eine im Wesentlichen konische Innenwand umfasst und das Spannfutter so angeordnet ist, dass es an der im Wesentlichen konischen Innenwand anliegt.

2. Verdampferinstrument nach Anspruch 1, wobei das Steuermittel (112) eine Rändelschraube ist, die mit dem Gehäuse (96) im Gewindeeingriff steht und mit dem Spannfutter (106) verbunden ist.

3. Verdampferinstrument nach Anspruch 1 oder Anspruch 2, wobei eine Öffnung in einem Ende des Gehäuses (96) zum Aufnehmen des Spiralstabs (114) vorgesehen ist.

4. Verdampferinstrument nach einem der Ansprüche 1 bis 3, wobei ein Spiralstabhalter (200) zur Verwendung mit dem Spiralstab und dem Spiralwerkzeug (94) vorgesehen ist, wobei der Spiralstabhalter eine Spiralstaböffnung (212) umfasst, die sich in einem Ende des Spiralstabhalters zum Aufnehmen des Spiralstabs (114) befindet.

5. Verdampferinstrument nach Anspruch 4, wobei ein Loch (208a) zum Führen des Spiraldrahts neben der Spiralstaböffnung (212) vorgesehen ist, und ein entsprechendes Loch (208b) an einer Seite oder einem anderen Ende des Spiralstabhalters (200) vorgesehen und mit dem Loch durch den Körper des Spiralstabhalters verbunden ist.

6. Verdampferinstrument nach Anspruch 5, wobei ein zweites Loch (210a) neben der Spiralstaböffnung (212) gegenüber dem ersten Loch (208a) vorgesehen ist, und ein zweites entsprechendes Loch (210b) an einer Seite oder einem anderen Ende des Spiralstabhalters (200) vorgesehen und mit dem zweiten Loch durch den Körper des Spiralstabhalters verbunden ist.

7. Verdampferinstrument nach einem der Ansprüche 4 bis 6, wobei die Spiralstaböffnung (212) einen im Wesentlichen sechseckigen oder nicht kreisförmigen Abschnitt zum Aufnehmen eines im Wesentlichen sechseckigen oder nicht kreisförmigen Endes des Spiralstabs (114) enthält.

8. Verdampferinstrument nach einem der vorhergehenden Ansprüche, wobei sich eine Drahtführung an einem Ende des Gehäuses (96) befindet, um bei Drehung des Spiralwerkzeugs (94) in Bezug auf den Spiralstab den Draht um den Spiralstab (114) herum zu führen, wobei die Drahtführung optional eine Schraube (116) ist.

9. Verdampferinstrument nach einem der vorhergehenden Ansprüche, wobei das Verdampferinstrument Teil eines Sets (10) ist, das eine Hülle (12) enthält, wobei die Hülle einen zentralen Körperabschnitt (14), eine obere Endkappe (16), die an einem Ende des zentralen Körperabschnitts befestigt werden kann, und entweder:
A) eine untere Endkappe (20), die am anderen Ende des zentralen Körperabschnitts befestigt werden kann; oder
B) eine Zwischenkappe (18), die am anderen Ende des zentralen Körperabschnitts befestigt werden kann, und eine untere Endkappe (20), die an der Zwischenkappe befestigt werden kann, aufweist.

10. Verdampferinstrument und -set nach Anspruch 9, umfassend die Elemente von (B), wobei eine Öffnung (92) in der Zwischenkappe (18) zum Aufnehmen eines Spiralstabs (114) vorgesehen ist.

11. Verdampferinstrument und -set nach Anspruch 10, wobei das Ende des Spiralstabs (114), das in der Zwischenkappe (18) aufgenommen ist, sechseckig ist und die Öffnung (92) in der Zwischenkappe entsprechend sechseckig ist.

12. Verdampferinstrument und -set nach einem der Ansprüche 9 bis 11, wobei ein Instrumentenzylinder (22) zum Aufbewahren von Instrumenten, einschließlich des Verdampferinstruments, sich innerhalb des zentralen Körperabschnitts (14) der Hülle (12) befindet.

13. Verdampferinstrument und -set nach einem der Ansprüche 9 bis 12, wobei sich eine Rolle (44) zum Aufbewahren von Dampfdraht (i) innerhalb des zentralen Körperabschnitts (14) und/oder der unteren Endkappe (20), wenn das Set die Elemente von (A) umfasst, oder (ii) innerhalb des zentralen Körperabschnitts und der Zwischenkappe (18) befindet, wenn das Set die Elemente von (B) umfasst.

14. Verdampferinstrument und -set nach Anspruch 13, wobei die Rolle (44) äußere Vorsprünge (47) aufweist, die um die Rolle herum beabstandet sind, um eine Wicklung des Dampfdrahts festzuhalten.

15. Verdampferinstrument und -set nach einem der Ansprüche 9 bis 14, wobei ein abdichtbarer Bereich zum Aufnehmen von Watte in der unteren Endkappe (20) ausgebildet ist, wobei der abdichtbare Bereich optional unterteilt ist, um einen Raum (84) für Watte und einen Raum (85) für Schrauben und vorgefertigte Verdampferspulen bereitzustellen.

## Revendications

1. Outil pour cigarette électronique pour la fabrication d'une bobine de vapotage comprenant une tige d'enroulement (114) et un outil d'enroulement (94) destiné à venir en prise avec et à tourner autour de la tige d'enroulement, l'outil d'enroulement ayant un carter (96) avec une paroi interne, une pince (106) disposée à l'intérieur du carter et agencée pour prendre appui contre la paroi interne, un diamètre intérieur de la pince étant déterminé par la position longitudinale de la pince à l'intérieur du carter et un moyen de commande (112) pour commander la position longitudinale de la pince à l'intérieur du carter, **caractérisé en ce que** la paroi interne comporte une paroi interne sensiblement conique, et la pince est agencée pour prendre appui contre la paroi interne sensiblement conique.

2. Outil pour cigarette électronique selon la revendication 1, dans lequel le moyen de commande (112) est une vis à oreilles mise en prise par filetage avec le carter (96) et reliée à la pince (106).

3. Outil pour cigarette électronique selon la revendication 1 ou 2, dans lequel une ouverture est prévue dans une extrémité du carter (96) pour recevoir la tige d'enroulement (114).

4. Outil pour cigarette électronique selon l'une quelconque des revendications 1 à 3, dans lequel un support de tige d'enroulement (200) est prévu pour être utilisé avec la tige d'enroulement et l'outil d'enroulement (94), dans lequel le support de tige d'enroulement comprend une ouverture de tige d'enroulement (212) disposée dans une extrémité du support de tige d'enroulement pour recevoir la tige d'enroulement (114).

5. Outil pour cigarette électronique selon la revendication 4, dans lequel un trou (208a) pour guider le fil d'enroulement est prévu à côté de l'ouverture de tige d'enroulement (212), et un trou correspondant (208b) est prévu sur un côté ou une autre extrémité du support de tige d'enroulement (200) et relié au trou à travers le corps du support de tige d'enroulement.

6. Outil pour cigarette électronique selon la revendication 5, dans lequel un deuxième trou (210a) est prévu de manière adjacente à l'ouverture de tige d'enroulement (212), à l'opposé du premier trou (208a), et un deuxième trou correspondant (210b) est prévu sur un côté ou une autre extrémité du support de tige d'enroulement (200) et relié au deuxième trou à travers le corps du support de tige d'enroulement.

7. Outil pour cigarette électronique selon l'une quelconque des revendications 4 à 6, dans lequel l'ouverture de tige d'enroulement (212) comporte une portion sensiblement hexagonale ou non circulaire pour recevoir une extrémité sensiblement hexagonale ou non circulaire de la tige d'enroulement (114).

8. Outil pour cigarette électronique selon l'une quelconque des revendications précédentes, dans lequel un guide-fil est disposé à une extrémité du carter (96), pour guider un fil autour de la tige d'enroulement (114) lors de la rotation de l'outil d'enroulement (94) par rapport à la tige d'enroulement, éventuellement dans lequel le guide-fil est une vis (116).

9. Outil pour cigarette électronique selon l'une quelconque des revendications précédentes, lequel outil pour cigarette électronique fait partie d'un kit (10) comportant un carter (12), le carter ayant une portion de corps centrale (14), un capuchon d'extrémité supérieur (16) pouvant être fixé à une extrémité de la portion de corps centrale, et soit :
A) un capuchon d'extrémité inférieur (20) pouvant être fixé à l'autre extrémité de la portion de corps centrale ; ou
B) un capuchon intermédiaire (18) pouvant être fixé à l'autre extrémité de la portion de corps centrale et un capuchon d'extrémité inférieur (20) pouvant être fixé au capuchon intermédiaire.

10. Outil et kit pour cigarette électronique selon la revendication 9, comprenant les éléments de (B), dans lesquels une ouverture (92) est prévue dans le capuchon intermédiaire (18) pour recevoir une tige d'enroulement (114) .

11. Outil et kit pour cigarette électronique selon la revendication 10, dans lesquels l'extrémité de la tige d'enroulement (114) reçue dans le capuchon intermédiaire (18) est hexagonale et l'ouverture (92) dans le capuchon intermédiaire est hexagonale de manière correspondante.

12. Outil et kit pour cigarette électronique selon l'une quelconque des revendications 9 à 11, dans lesquels un barillet d'outil (22) pour stocker des outils comportant l'outil pour cigarette électronique est disposé à l'intérieur de la portion de corps centrale (14) du carter (12) .

13. Outil et kit pour cigarette électronique selon l'une quelconque des revendications 9 à 12, dans lesquels une bobine (44) pour stocker le fil de vapotage est disposée (i) à l'intérieur de la portion de corps centrale (14) et/ou du capuchon d'extrémité inférieure (20) si le kit comprend les éléments de (A), ou (ii) à l'intérieur de la portion de corps centrale et du capuchon intermédiaire (18) si le kit comprend les éléments de (B).

14. Outil et kit pour cigarette électronique selon la revendication 13, dans lesquels la bobine (44) présente des saillies externes (47) espacées autour de la bobine pour retenir un enroulement de fil de vapotage.

15. Outil et kit pour cigarette électronique selon l'une quelconque des revendications 9 à 14, dans lesquels une zone scellable destinée à contenir du coton est formée à l'intérieur du capuchon d'extrémité inférieur (20), facultativement dans lesquels la zone scellable est divisée pour fournir un espace (84) pour le coton et un espace (85) pour les vis et les bobines de vapotage préfabriquées.
